# EUROPEAN PATENT SPECIFICATION

(11) **EP 0 151 492 B1**
(45) Date of publication and mention of the grant of the patent: **17.01.1996**
(21) Application number: 85101353.2
(22) Date of filing: 08.02.1985
(51) Int. Cl.: G01N 33/535, C12Q 1/68, G01N 33/53

(54) **Heterologous system for the detection of labeled DNA**
Heterologes System zum Nachweis markierter DNA
Système hétérologue pour la détection d'ADN marqué

(30) Priority: 09.02.1984 US 578732
(43) Date of publication of application: 14.08.1985
(73) Proprietor: ENZO BIOCHEM, INC., New York, N.Y. 10013 (US)
(72) Inventor: Rabbani, Elazar, New York, N.Y. 10003 (US)
(74) Representative: VOSSIUS & PARTNER

(56) References cited:
- EP-A- 0 063 879
- EP-A- 0 122 614
- EP-A- 0 123 300
- EP-A- 0 128 332
- WO-A-84/04970
- WO-A-85/03356

## Description

The present invention relates to a composition for detecting the presence or absence of an analyte in a sample.

The detection of labeled molecules, such as chemically-labeled DNA or other materials or molecules, containing a receptor or target moiety thereon, either naturally-occurring or placed thereon, is an important activity from a commercial, diagnostic and scientific point of view. For example, biotinylated nucleotides capable of being incorporated into double-stranded DNA have been prepared. Single-stranded DNA containing such biotinylated nucleotides have been employed, after hybridization with complementary single-stranded DNA, to identify various types of DNA material. The presence of the biotinylated nucleotide in the hybridized double-stranded DNA is generally detected by a reaction involving the affinity between biotin and avidin, the avidin normally attached to a biotinylated enzyme, such as biotinylated horseradish peroxidase. Streptavidin may be substituted for avidin in such a system. The utilization of streptavidin or avidin as a probe for the detection of biotinylated nucleotides or biotinylated DNA is disclosed in co-owned European Patent Application Publication No. 0 063 879 published March 11, 1983.

It has also been proposed to label nucleotides and DNA with glycosyl groups or sugar moieties, such as maltose, lactose, mannose, triose, and to detect the thus-labeled nucleotides or DNA with a lectin, such as Concanavalin A, which possesses a strong affinity for sugar groups and glycoproteins. The labeling of DNA with glycosyl groups and detection of the thus-labeled DNA is disclosed in EP-A 097 373.

Other techniques for detection of biological materials have also been proposed. For example, European Patent Application Publication No. 0 071 976 published February 16, 1983 involves the covalent binding of biotin to immunologically active material and covalent binding of avidin to an enzyme, such as horseradish peroxidase. Another detection technique is disclosed in European Patent Application Publication No. 0 074 520 published March 23, 1983. This European patent application publication discloses a technique for the detection of human chorionic gonadotropin (HCG) involving a lectin bound to a solid support brought into contact with a sample, such as a urine specimen, suspected of containing HCG. After removal from the support, the resulting lectin-fixed HCG is then brought into contact with an antibody and a color carrier material. The presence of lectin-fixed HCG is indicated by color formation.

It is an object of this invention to provide improved techniques and materials useful in the identification and/or determination of an analyte.

In accordance with the practices of this invention, there is provided a heterologous detection system and components useful in conjunction therewith and kits for carrying out the heterologous detection system. The heterologous detection system employs the affinity between avidin or streptavidin and biotin, along with the affinity between a lectin and a glycoprotein and/or a glycosyl or sugar group. Particularly useful components in the practices of this invention are the biotinylated lectins and biotinylated sugars (particularly biotinylated polysaccharides, such as biotinylated dextran), biotinylated glycoproteins, and biotinylated enzymes.

The present invention relates to a composition for detecting the presence or absence of an analyte in a sample, which composition comprises:
(a) a biotinylated polynucleotide specifically bindable with said analyte;
   a heterologous entity comprising a first member selected from avidin or streptavidin, said first member having affinity for said biotinylated polynucleotide, and as a second member a lectin, a glycoprotein, or a glycosyl- or a sugar-containing compound, and
   a signalling entity comprising as a third member a lectin and attached thereto, a signal generating portion comprising an enzyme and capable of forming a signal associated with detection of said analyte in the instance where the second member comprises a glycoprotein, or a glycosyl- or a sugar-containing compound and said third member comprises a glycoprotein or a glycosyl-or sugar-containing compound and attached thereto, a signal generating portion comprising an enzyme and capable of forming a signal associated with detection of said analyte or comprises an enzyme containing a glycosyl group in the instance where the second member comprises a lectin, said third member having affinity for said second member; or
(b) a glycosylated polynucleotide specifically bindable with said analyte;
   a heterologous entity comprising as a first member a lectin, said first member having affinity for said glycosylated polynucleotide, and as a second member biotin, avidin or streptavidin; and
   a signalling entity comprising as a third member biotin in the instance where the second member comprises avidin or streptavidin, and said third member comprises avidin or streptavidin in the instance where the second member comprises biotin, said third member having affinity for said second member and, attached thereto, a signal generating portion comprising an enzyme and capable of forming a signal associated with detection of said analyte.

Avidin is a glycoprotein having a molecular weight of about 68,000 and a very high affinity for biotin, greatly exceeding that of an antibody for most antigens. Specifically, an avidin molecule provides four binding sites for a biotin molecule. Avidin-biotin affinity is essentially irreversible and comparable to a covalent bond. Proteins, glycoproteins, and enzymes can be conjugated with several molecules of biotin. The special affinity between avidin and biotin provides for the possibility of forming macromolecular complexes between avidin and avidin-containing materials and biotin-containing materials, such as biotinylated enzymes, lectins and polysaccharides. Streptavidin, a molecule closely related to avidin, is preferably substituted for avidin in such complexes, particularly in connection with the detection of biotin-labeled DNA or glycosylated DNA.

Lectins and biotinylated lectins are an important component of the heterologous detection system in accordance with the practices of this invention. Lectins are proteins or glycoproteins with two or more binding sites that recognize a specific sequence of sugar residues. Although originally isolated from plants, lectins have been found in all types of organisms. Lectins which are usefully employed in the practices of this invention include dolichos biflorus agglutinin, which has specificity for the group N-acetylgalactosaminyl, lentil lectin which has an affinity for alpha-D-mannose and alpha-D-glucose, as does garden pea lentil lectin. Many other lectins are known and are commercially available. Several commercially available lectins and the specific sugar residues they recognize are set forth in accompanying Table I:

**TABLE I**

| Lectin | Sugar Specificity |
|---|---|
| Concanavalin A (from Jack beans) | alpha-D-glucose and alpha-D-mannose |
| Soybean Lectin | D-galactose and N-acetyl |
| | D-galactosamine |
| Wheat Germ Lectin | N-acetylglucosamine |
| Lotus Seed Lectin | fucose |
| Potato lectin | |

Also of interest in the practices of this invention are glycolipids which are oligosaccharide-containing molecules found on the surface of all plasma membranes. More specifically, glycolipids are present in the outer half of the bilayer of the cell membrane with the sugar group exposed on the cell surface. The neutral glycolipids with polar head groups consisting of from 1-15 or more neutral sugars are found widely distributed in the plasma membranes of both eucaryotic and procaryotic cells. Some glycolipids are found only in certain mammals and usually in only certain tissues thereof. For example, galactocerebroside, one of the simplest glycolipids containing only galactose and its polar head group, is the main glycolipid in myelin. More complex glycolipids are the gangliosides of which about 30 have been identified. Lectins would be useful for attachment to the exposed glycolipid oligosaccharide groups as carriers or substrates for cell membrane identification because of the normal affinity between lectins and sugar groups.

Of special interest in the practices of this invention are the glycoproteins, particularly the enzymes, such as glucose oxidase, peroxidase, horseradish peroxidase, alkaline phosphatase, acid phosphatase and β-galactosidase. The above-mentioned enzymes are merely exemplary of the many known glycoproteins or enzymes, which are useful in the practices of this invention.

One aspect of the practices of this invention involves biotinylated enzymes. Various techniques may be employed in the preparation of biotinylated enzymes. An exemplary preferred technique is disclosed in EP-A 123 300. This application discloses a technique for the biotinylation of enzymes wherein the enzyme is fixed to a solid substrate, preferably through a competitive inhibitor therefor, which protects the active site of the enzyme during the exposure of the enzyme to biotin. After the enzyme has been biotinylated, it is released from attachment to the competitive inhibitor which remains fixed to the solid substrate. Alternatively, while the biotinylated enzyme is still fixed to the solid substrate, further reaction or complex formation such as the addition of avidin or streptavidin to the fixed biotinylated enzyme, can be performed. After the attachment of avidin or streptavidin to the fixed biotinylated enzyme, the resulting complex can be released from the solid substrate. By employing this technique, various complexes can be prepared. For example, the fixed enzyme can be bound to a lectin rather than to biotin with the resulting formation of an enzyme-lectin complex which can then be removed from attachment to the solid substrate and employed as the reagent in the heterologous system in accordance with this invention. This technique is also applicable for the preparation of biotinylated lectins and biotinylated polysaccharides, among others. These products, as well as the enzyme lectin complex, can be employed to produce other larger complexes including other enzymes, glycoproteins and the like.

Techniques for the employment of an enzyme complex in the identification of a labeled DNA are disclosed in EP-A 124 124. This patent application describes techniques for the analysis of genetic material, such as DNA and RNA. The genetic material to be analyzed or identified is denatured, fixed to a substrate and hybridized with a probe, such as a chemically-labeled probe having a nucleotide sequence complementary to the genetic material to be identified. After the hybridization between the chemically-labeled probe and the DNA to be identified, an enzyme component effective on contact with a chromogen to produce an insoluble color precipitate or color product is attached to the probe. These techniques are applicable to the heterologous detection system in accordance with this invention and provide for the analysis and detection of genetic material with improved sensitivity, definition, accuracy and/or speed. The heterologous detection system of this invention is especially useful in such well known analytical DNA techniques, such as Southern blot analysis, Northern blot analysis, Western blot analysis, colony hybridization, plaque lifts, cytoplasmic dot hybridization and other analytical techniques for for the identification of genetic material such as DNA and RNA.

The following examples indicate the practices of this invention and are directed to the detection of various materials, such as human chorionic gonadotropin and DNA. In the tests employing biotinylated DNA and glycosylated DNA, the labelled DNA is hybridized to its complementary DNA utilizing a blot test technique in which the hybridized DNA is fixed to a suitable substrate. Example 1 illustrates a non-claimed use of a heterologous detection system including the use of antibodies to a specific biological material in fixing the material to a substrate, the use of a lectin capable of attachment to the biological material and the use of a signal moiety specific for the lectin.

### Example No. 1

Monoclonal antibodies to human chorionic gonadotropin (HCG) were bound to dextran (Sepharose®) beads employing cyanogen bromide. In addition to its use as a coupling agent, the cyanogen bromide destroys most of the affinity of the Sepharose® for the lectin Concanavalin A. Glass beads or polyacrylamide beads could be used in place of dextran beads to minimize control levels of Concanavalin A binding.

250 µl of the Sepharose® beads were incubated with 20 µl (∼670 units) of a preparation containing HCG for about 45 minutes at room temperature. In the control test no hormone was added or present. The Sepharose® beads were washed five times with 2.5 ml aliquots of phosphate buffered saline (PBS). After each washing the beads were separated by centrifugation at ∼ 1000 xg for one minute. The supernatant liquids were discarded and the beads were then washed once with 2 ml of 0.2 M imidazole buffer, pH.6.8, 1 mM Mn++, 1 mM Ca++. The beads were again separated by centrifugation. The imidazole Mn-Ca buffer was selected to maximize Concanavalin A binding.

The beads were then treated with 50 g of iodinated Concanavalin A, specific activity of 6400 cpm/ug in 400 µl of 0.2 M imidazole buffer, ph 6.8, 1 mM Mn++ and 1 mM Ca++. Following incubation for about 30 minutes at room temperature, the samples were washed three times with 5 ml aliquots of 0.2 M NaCl. The radioactivity in the supernatant from the last wash was approximately 67 cpm/100 µl. The beads were then separated by centrifugation, suspended in 0.2 M NaCl and counted. For the sample containing HCG, 231,840 cpm were bound and for the control, 117,150 cpm were bound.

In addition to the radioactive determination of the bound Concanavalin A, the presence of Concanavalin A bound to the HCG affixed to the beads could be determined in accordance with the practices of this invention by adding and fixing an enzyme to the bound Concanavalin A. The presence of the thus-bound enzyme could be determined or evidenced by conventional means, i.e., by providing a suitable enzyme chromogen-containing substrate for reaction with the Concanavalin A bound enzyme. In the above example a heterologous detection system is employed involving antibodies for linking with the HCG, the HCG linked to the lectin, Concanavalin A, and the lectin linked to a glycoprotein, the enzyme.

In the above example the determination of the amount of bound Concanavalin A would detection with streptavidin-biotinylated horseradish peroxidase enzyme complex. In accordance with this aspect of the heterologous detection system of this invention, the streptavidin attaches itself to the Concanavalin A along with the biotinylated horseradish peroxidase and the thus-fixed streptavidin biotinylated horseradish peroxidase is evidenced by a suitable chromogen or chromogenic or color yielding reaction involving the fixed horseradish peroxidase.

### Example No. 2

A complex formed by biotinylated DNA and streptavidin-biotin horseradish peroxidase allows the detection of 100 pcg of DNA bound to nitrocellulose paper. This sensitivity of biotinylated DNA with streptavidin-biotin horseradish peroxidase is indicated in the above-identified EP-A 124 124. Glycosylated DNA and lectin for the determination of the glycosylated DNA is not as sensitive. The use of the lectin system for the detection of glycosylated DNA is advantageous, however, since the enzyme, being also glycosylated, can be directly bound to the glycosylated DNA-lectin without further manipulation. The special component of this invention, biotinylated lectin, offers a special technique in accordance with the practices of this invention to improve the glycosylated DNA-lectin system for the detection of DNA.

DNA dot blots were prepared using naturally glycosylated DNA (T₄ Phage DNA) and nick translated biotinylated DNA. The nitrocellulose or similar filters containing the DNA dot blots were blocked to prevent non-specific binding of the lectin, Concanavalin A, thereto. This procedure involves blocking at about 50°C. with 2% BSA, washing once with SSC and contacting with 0.1% of the surfactant Triton X-100. The DNA dot blots were then processed.

The DNA dot blots were contacted with complementary DNA which had been biotinylated to about 20% biotinylation by nick translation. These tests are indicated in Table II, below.

**TABLE II**

| Biotinylated DNA | Carrier DNA |
|---|---|
| 2 ng | 4.5 ng |
| 1 ng | 4.5 ng |
| 500 pcg | 4.5 ng |
| 250 pcg | 4.5 ng |
| 125 pcg | 4.5 ng |
| 62.5 pcg | 4.5 ng |
| 31.25 pcg | 4.5 ng |
| 15.625 pcg | 4.5 ng Control |

For comparison the streptavidin-biotin horseradish peroxidase complex was added, after blocking, in the amount 20 µl/cm to the biotinylated DNA blots and incubated at 37°C. for 45 minutes. The blots were rinsed three times at 5 minutes each in a high salt buffer and two times at 5 minutes each in a low salt buffer at room temperature. Thereupon, DAB (0.5 mg/ml in 10 mM Tris at a pH 7.6 plus 0.02% CoCl₂) was applied for 10 minutes at about 0°C. in the dark. Afterwards, 0.02% H₂O₂ was added for immediate detection of the complex.

The glycosylated DNA blots employing the glycosylated T₄ phage DNA were tested as indicated in the following Table III.

**TABLE III**

| T₄ phage DNA | Carrier DNA |
|---|---|
| 500 pcg | 4.5 ng |
| 250 pcg | 4.5 ng |
| 125 pcg | 4.5 ng |
| 62.5 pcg | 4.5 ng |
| 31.25 pcg | 4.5 ng |
| 15.625 pcg | 4.5 ng |
| 7.8125 pcg | 4.5 ng |
| 3.9 pcg | 4.5 ng |
| 0 | Control |

After blocking of the blots, biotinylated Concanavalin A was applied to the strip of blots in the amount of 20 ul/cm or 100 ug/ml in TCMN solution made up of 5 mM Tris pH 7.0, 1 mM MnCl₂, 1 mM CaCl₂ and 100 mM Nacl and the blots were incubated at 37°C. for 1 hour in a damp atmosphere. The experimental results indicated that rinsing with TCMN buffer (100-200 ml) resulted in high background (non-specific binding of Concanavalin A to the nitrocellulose paper).

Accordingly, 1 mM glucose solution was substituted for the wash buffer in another set of experiments. In this set of experiments the glycosylated DNA blots on nitrocelllose filter paper were blocked at 50°C. with 2% BSA, washing once with SSC and 0.1% surfactant, Triton X-100®. Biotinylated Concanavalin A in the amount 100 ug/ml or 20 ul/cm in TCMN buffer was applied at 37°C. for 60 minutes, followed by five rinses or soakings of 5 minutes each with 1 mM alpha-D-glucose and three soakings or rinses at 5 minutes each with TCMN. The streptavidin-biotin-horseradish peroxidase complex was then applied at 37°C for 30 minutes and washed with a high salt buffer and a low salt buffer, followed by detection of the blot employing DAB-H₂O₂, as described hereinabove. The observed results indicated a greatly increased sensitivity for the detection of the glycosylated DNA.

The above examples illustrate the versatility and broad applicability of the practices of the heterologous detection system of this invention, which does not depend upon only one ligand or reaction or affinity. Where a target material has attached thereto a biotin moiety, a homologous detection system is known which involves an enzyme complex comprising avidin or streptavidin-biotin enzyme, e.g., streptavidin-biotin horseradish peroxidase. Where the target material is glycosylated DNA, another homologous detection system is known involving lectin for attachment to the glycosylated DNA or other sugar groups attached to the target material, and an enzyme or enzyme complex capable of attachment to the lectin. The lectin joins both the target moiety, glycosylated DNA, and the signal moiety comprising the enzyme.

In the heterologous detection system in accordance with this invention, however, a preferred embodiment thereof resides in the use of the combination involving the affinity between biotin and avidin or biotin and streptavidin and the affinity between a lectin and a glycoprotein. This heterologous detection system in effect unexpectedly combines the features of two homologous detection systems. For example, when the target contains biotin groups, such as biotinylated DNA, the detection system would employ a complex, which contains avidin or streptavidin and biotinylated lectin. This complex would fix or attach itself to the biotin portion of the target and would be elicited by bringing into contact therewith and fixing to the lectin portion of the complex an enzyme containing a glycosyl group, such as horseradish peroxidase. This enzyme would then be activated or employed to signal its attachment to the avidin biotinylated lectin complex. Additionally, the avidin biotinylated lectin complex could be elicited by contact with the enzyme complex, streptavidin-biotinylated enzyme, such as streptavidin-biotin horseradish peroxidase.

For the determination of a glycosylated or sugar-containing target material, biotinylated lectins could be attached directly thereto, the biotinylated lectin bound to the target material are brought into contact with an enzyme complex, e.g., streptavidin-biotin horseradish peroxidase. This system would provide two signals, one effected by the attachment of the enzyme directly to the lectin and the other by the attachment of the streptavidin-biotin enzyme complex to the biotin moiety of the biotinylated lectin.

The applicability of the biotinylated lectins and biotinylated enzymes in the practices of heterologous detection system of this invention has been indicated above. Another special component, a biotinylated polysaccharide, such as biotinylated dextran or biotinylated agarose, also provides advantages in the practices of this invention. For example, in the detection of a sugar-labelled target material, a lectin would be brought into contact with the glycosylated DNA for attachment thereto. Biotinylated dextran would then be brought into contact with the lectin, now fixed to the glycosylated DNA. The biotinylated dextran attached to the target-fixed lectin could be elicited by contact with a subsequently added lectin enzyme complex and by contact with an avidin or streptavidin-biotin-enzyme complex. The avidin or streptavidin-biotin complex would attach itself to the biotin moiety of the biotinylated dextran. Because many sites are offered by the biotinylated dextran for attachment with the probe or signal-generating lectin-enzyme complex and/or avidin or streptavidin-biotin enzyme complex, a substantial signal amplification or enlargement is obtainable.

In accordance with the practices of this invention, therefore, signal enlargement or amplification is obtainable by employing in combination a biotinylated lectin, a biotinylated dextran for attachment thereto and for further attachment of a lectin complex, or an avidin or streptavidin enzyme complex or an avidin-biotin dextran complex with eventual utilization of a lectin-enzyme complex or a streptavidin or avidin-biotin enzyme complex. There may also be employed immunobiologically active materials, such as antigens, antibodies and anti-antibodies, in the combination heterologous detection system of this invention, see for example, U.S. Patent 4,289,747.

## Claims

1. A composition for detecting the presence or absence of an analyte in a sample, which composition comprises:
(a) a biotinylated polynucleotide specifically bindable with said analyte;
a heterologous entity comprising a first member selected from avidin or streptavidin, said first member having affinity for said biotinylated polynucleotide, and as a second member a lectin, a glycoprotein, or a glycosyl- or a sugar-containing compound, and
a signalling entity comprising as a third member a lectin and attached thereto, a signal generating portion comprising an enzyme and capable of forming a signal associated with detection of said analyte in the instance where the second member comprises a glycoprotein, or a glycosyl- or a sugar-containing compound and said third member comprises a glycoprotein, or a glycosyl- or a sugar-containing compound and attached thereto, a signal generating portion comprising an enzyme and capable of forming a signal associated with detection of said analyte or comprises an enzyme containing a glycosyl group in the instance where the second member comprises a lectin, said third member having affinity for said second member; or
(b) a glycosylated polynucleotide specifically bindable with said analyte;
a heterologous entity comprising as a first member a lectin, said first member having affinity for said glycosylated polynucleotide, and as a second member biotin, avidin or streptavidin; and
a signalling entity comprising as a third member biotin in the instance where the second member comprises avidin or streptavidin, and said third member comprises avidin or streptavidin in the instance where the second member comprises biotin, said third member having affinity for said second member and, attached thereto, a signal generating portion comprising an enzyme and capable of forming a signal associated with detection of said analyte.

2. The composition (a) of claim 1 wherein said signalling entity comprises either a biotinylated enzyme or a biotinylated enzyme complexed with avidin or streptavidin.

3. The composition of claim 1 wherein said enzyme is selected from glucose oxidase, peroxidase, horseradish peroxidase, alkaline phosphatase, acid phosphatase, β-galactosidase, chloroperoxidase and monoamine oxidase.

4. The composition of claim 1 wherein said lectin is selected from Concanavalin A, soybean lectin, wheatgerm lectin, ulex europaeus agglutinin I, peanut agglutinin, ricinus communis agglutinin I, phaseolus vulgaris agglutinin, japonica agglutinin, lotus seed lectin, potato lectin, dilichos biflorus agglutinin and a lentil lectin, or a combination of any of the foregoing.

## Patentansprüche

1. Mittel zum Nachweis des Vorhandenseins oder Nichtvorhandenseins eines Analyten in einer Probe, wobei das Mittel folgende Bestandteile umfaßt:
(a) ein biotinyliertes Polynucleotid, das spezifisch mit dem Analyten binden kann;
eine heterologe Einheit, die eine erste Komponente umfaßt, ausgewählt aus Avidin oder Streptavidin, wobei die erste Komponente eine Affinität für das biotinylierte Polynucleotid aufweist, und als zweite Komponente ein Lectin, ein Glykoprotein oder eine einen Glykosylrest oder einen Zucker enthaltende Verbindung, und
eine Signaleinheit, die in dem Fall, in dem die zweite Komponente ein Glykoprotein oder eine einen Glykosylrest oder einen Zucker enthaltende Verbindung umfaßt, als dritte Komponente ein Lectin umfaßt und damit verknüpft eine ein Enzym umfassende signalerzeugende Einheit, die fähig ist zur Erzeugung eines Signals, das mit dem Nachweis des Analyten in Zusammenhang steht, und wobei die dritte Komponente ein Glykoprotein, eine einen Glykosylrest oder einen Zucker enthaltende Verbindung umfaßt und damit verknüpft eine ein Enzym umfassende signalerzeugende Einheit, die fähig ist zur Erzeugung eines Signals, das mit dem Nachweis des Analyten in Zusammenhang steht, oder ein einen Glykosylrest enthaltendes Enzym umfaßt, in dem Fall, in dem die zweite Komponente ein Lectin umfaßt, wobei die dritte Komponente eine Affinität für die zweite Komponente aufweist; oder
(b) ein glykosyliertes Polynucleotid, das spezifisch mit dem Analyten binden kann;
eine heterologe Einheit, die als erste Komponente ein Lectin umfaßt, wobei die erste Komponente eine Affinität für das glykosylierte Polynucleotid aufweist, und als zweite Komponente Biotin, Avidin oder Streptavidin; und
eine signalerzeugende Einheit, die in dem Fall als dritte Komponente Biotin umfaßt, in dem die zweite Komponente Avidin oder Streptavidin umfaßt, und wobei die dritte Komponente dann Avidin oder Streptavidin umfaßt, wenn die zweite Komponente Biotin umfaßt, wobei die dritte Komponente eine Affinität für die zweite Komponente aufweist, und damit verknüpft eine ein Enzym umfassende signalerzeugende Einheit, die fähig ist zur Erzeugung eines Signals, das mit dem Nachweis des Analyten in Zusammenhang steht.

2. Mittel (a) nach Anspruch 1, wobei die Signaleinheit entweder ein biotinyliertes Enzym oder ein biotinyliertes Enzym im Komplex mit Avidin oder Streptavidin umfaßt.

3. Mittel nach Anspruch 1, wobei das Enzym ausgewählt ist aus Glucoseoxidase, Peroxidase, Meerrettichperoxidase, alkalischer Phosphatase, saurer Phosphatase, β-Galactosidase, Chlorperoxidase und Monoaminoxidase.

4. Mittel nach Anspruch 1, wobei das Lectin ausgewählt ist aus Concanavalin A, Sojabohnenlectin, Weizenkeimlectin, Ulex europaeus-Agglutinin I, Erdnußagglutinin, Ricinus communis-Agglutinin I, Phaseolus vulgaris-Agglutinin, Japonica-Agglutinin, Lotussamenlectin, Kartoffellectin, Dilichos biflorus-Agglutinin und einem Linsenlectin oder einer Kombination der vorstehend beschriebenen Komponenten.

## Revendications

1. Composition pour détecter la présence ou l'absence d'un analyte dans un échantillon, ladite composition comprend:
(a) un polynucléotide biotinylé pouvant se lier spécifiquement avec ledit analyte;
une entité hétérologue comprenant un premier élément choisi parmi l'avidine ou la streptavidine, ledit premier élément ayant une affinité pour ledit polynucléotide biotinylé, et comme second élément une lectine, une glycoprotéine ou un composé contenant un glycosyle ou un sucre,
une entité de signalisation comprenant comme troisième élément une lectine et, fixée à celle-ci, une portion engendrant un signal comprenant une enzyme et capable de former un signal associé à la détection dudit analyte dans le cas où le second élément comprend une glycoprotéine ou un composé contenant un glycosyle ou un sucre et ledit troisième élément comprend une glycoprotéine ou un composé contenant un glycosyle ou un sucre et, fixée à celui-ci, une portion engendrant un signal comprenant une enzyme et capable de former un signal associé à la détection dudit analyte ou qui comprend une enzyme contenant un groupe glycosyle dans le cas où le second élément comprend une lectine, ledit troisième élément ayant une affinité pour ledit second élément; ou
(b) un polynucléotide glycosylé pouvant se lier spécifiquement avec ledit analyte;
une entité hétérologue comprenant comme premier élément une lectine, ledit premier élément ayant une affinité pour ledit polynucléotide glycosilé, et en tant que second élément la biotine, l'avidine ou la streptavidine; et
une entité de signalisation comprenant comme troisième élément la biotine dans le cas où le second élément comprend l'avidine ou la streptavidine, et ledit troisième élément comprend l'avidine ou la streptavidine dans le cas où le second élément comprend la biotine, ledit troisième élément ayant une affinité pour ledit second élément et, fixée à celle-ci, une portion engendrant un signal comprenant une enzyme et capable de former un signal associé avec la détection dudit analyte.

2. Composition (a) selon la revendication 1, dans laquelle ladite entité de signalement comprend soit une enzyme biotinylée soit une enzyme biotinylée complexée avec l'avidine ou la streptavidine.

3. Composition selon la revendication 1, dans laquelle ladite enzyme est choisie parmi la glucose oxydase, la peroxydase, la peroxydase de raifort, l'alcaline phosphatase, l'acide phosphatase, la β-galactosidase, la chloroperoxydase et la monoamine oxydase.

4. Composition selon la revendication 1, dans laquelle ladite lectine est choisie parmi la Concanavalin A, la lectine de soja; la lectine de germe de blé, l'agglutinine I de ulex europaeus, l'agglutinine d'arachide, l'agglutinine I de Ricinus communis, l'agglutinine de phaseolus vulgaris, l'agglutinine japonica, la lectine de lotus, la lectine de pomme-de-terre, l'agglutinine de dilichos biflorus et une lentilectine, ou une combinaison de tout élément ci-dessus.
